# EUROPEAN PATENT APPLICATION

(11) **EP 1 138 288 A1**
(43) Date of publication of application: **04.10.2001**
(21) Application number: 01200998.1
(22) Date of filing: 19.03.2001
(51) Int. Cl.: A61F 5/01

(54) **Ankle support**

(30) Priority: 24.03.2000 NL 1014740
(71) Applicant: Nea International B.V., 6224 LV Maastricht (NL)
(72) Inventor: Voskuilen, Agnes Theodora Maria, 6229 TB Maastricht (NL)
(74) Representative: Krijgsman, Willem

(57) **Abstract**

Ankle support having a lateral side and a medial side, at least comprising
- an elongate support element (18), which runs on the medial side, has a bottom section (22) and a top section (24) and is anatomically adapted to the lower leg (10), the ankle and the foot, which support element (18) is provided with means for fixing it against movement in its longitudinal direction, wherein the ankle support also comprises:
- a securing element (4) which runs on the lateral side;
- at least two draw strips (8, 12) which, when the ankle support is arranged on the foot, each run along different sides of the lower leg (10) and connect the top section (24) of the support element (18) to the securing element (4).

## Description

The invention relates to an ankle support having a lateral side and a medial side, at least comprising a flexible, elongate support element which runs on the medial side and has a bottom section and a top section, which support element is anatomically adapted to the lower leg, the ankle and the foot.

In certain cases, such as in the case of a foot which has a natural tendency to be sprained and in sports which impose a high load on the ankle, the stability and protection against the foot being sprained by turning inwards, with the ankle joint being inverted, is insufficient. As a result, the foot can turn inwards, which may cause an inversion trauma. The inversion trauma leads to damage to various joint structures, which is generally cured only by extensive and prolonged medical and/or physiotherapy treatment.

To prevent inversion from occurring, various ankle supports have already been proposed and are in use. In many cases, they comprise shells which enclose a section of the lower leg and sometimes also a part of the foot and are secured tightly around the leg and foot using strips or laces. Ankle supports of this type are known, for example, from DE-G-94 20 046.7, EP-A-275,543 and EP-A-305,999. Ankle supports of this type have the drawback that the freedom of movement of the foot is also impeded in directions other than that in which inversion occurs.

EP-A-372,452 has disclosed a U-shaped ankle support which encloses only a small part of the foot and therefore provides less of an obstacle to the mobility of the foot in directions in which no fixing is required. The support is attached by two strips which are wound around the lower leg. In this support, however, the foot also has sufficient space in the direction of inversion for there still to be a possibility of injury in the event of the foot spraining under a heavy load.

WO-98/29060 has disclosed a medium-weight ankle support which comprises two parts which lie against the foot, on which one part is a supporting element as described in the preamble. The two parts together form a likewise U-shaped ankle support, of which the lateral section is more flexible that the medial section. In this support too, there is still a certain risk that, in the event of an inversion occurring, the support will be displaced with respect to the foot and lower leg, with the result that the load on the ankle will not be fully absorbed. The application does not describe how the ankle support is to be secured to the leg in order possibly to prevent this.

It is now an object of the invention to provide an ankle support which is better able to prevent inversion of the foot than the known ankle support.

According to the invention, this object is achieved by the fact that the ankle support also comprises a securing element which runs on the lateral side and at least two draw strips which, when the ankle support is arranged on the foot, each run along different sides of the lower leg and connect the top section of the support element to the securing element.

The ankle support according to the invention ensures that in the event of even the slightest inversion of the foot the draw strips, in conjunction with the support element, immediately and almost completely prevent further movement in the direction associated with the inversion. The movement associated with the inversion is translated into the application of a tensile load to the draw strips, which directly transmit this load to the support element. The course of the draw strips imposes a load on the support element in its longitudinal direction and the support element is fixed against movement in this direction.

A further advantage of the ankle support according to the invention is that substantially no further features are required for securing the ankle support to the foot, as is the case in the known ankle support. The structure is therefore lightweight, not very bulky and does not limit or scarcely limits the freedom of movement in directions other than that of an inversion.

The support element is elongate and, with the ankle support fitted, runs virtually vertically from a position which lies below the medial malleolus, upwards along the medial malleolus and then along a part of the lower leg. The longitudinal direction is intended to mean the direction defined in this way. A support element is flexible if it is sufficiently deformable perpendicular to the longitudinal direction to anatomically adapt itself to the contours of that section of the foot and leg along which it runs, but also does not bend in its longitudinal direction when a load is imposed on the support element by the draw strips. Suitable support elements can be made from metal or plastic. It also leaves the medial malleolus free during normal walking movements. To this end it may, for example, be narrow and run along the malleolus, but may also be wider, in which case it can be provided with an opening which exposes the malleolus. The support element is provided with means for fixing it against movement in its longitudinal direction, which means will be described in more detail below. At the top section, there are features by means of which the draw strips can be connected to the support element.

On the lateral side, the ankle support has a securing element. This element serves to fix the draw strips, which are likewise present, on the lateral side of the foot. The securing element is connected at the foot, below the lateral malleolus, and preferably also extends over an adjoining section, which lies further forwards, of the lateral side of the foot. For optimum freedom of movement of the foot, it is preferably made from leather or a flexible plastic, but may also be designed as a shell of a harder material which matches the shape of the foot. In any case, the securing element, like the draw strips, must be completely or almost completely free of elongation under tensile load.

It must be possible to fix the securing element with respect to the foot. A closure strip which runs over the instep and further around the foot and which connects two opposite sides of the securing element to one another is eminently suitable for this purpose.

A further improved fixing of the securing element can be obtained by not only connecting it at the lateral side of the foot but also making it run from this lateral side under at least part of the sole of the foot. The closure strip then runs only over the instep.

The draw strips are connected to the securing element at a location which lies lower than the lateral ankle joint. As a result, in the event of inversion of the foot, a downward pulling movement is exerted on the draw strips, with the beneficial effect described above.

In a preferred embodiment of the ankle support according to the invention, the closure strip and one of the draw strips form a single unit and the securing element is provided with an opening for the combined closure-draw strip to pass through. The draw strip which has been lengthened in this way is then preferably securely connected to the securing element underneath or close to the sole of the foot, while its other end is free. When the ankle support is being fitted, the free end of the strip coming from the sole of the foot is then guided over the instep, via the medial side of the foot, is pulled through the opening in the securing element and, from there, is turned back and guided along the front of the lower leg, obliquely upwards, towards the top section of the support element. The free end of the strip is then connected to the top section of the support element. In this way, the strip combines the function of a closure strip which fixes the securing element and of a draw strip in one.

Closure and draw strips should consist of a material without elasticity or with very low elasticity, in order to avoid the possibility of the intended effect, i.e. of preventing inversion of the foot, being lost as a result of elasticity in the strips. The permissible elasticity is determined in connection with the length of the strips, by the movement which is still permissible in the event of inversion of the ankle joint without an undesirable or unacceptable trauma occurring.

The draw strips are both connected to the securing element at a height which lies below the height of the lateral malleolus. From there, they each run a different distance along the lower leg towards the support element. In this case, one of the draw strips runs along the back side of the lower leg and another draw strip runs along the front side of the lower leg. The draw strips therefore run obliquely upwards from a position which lies lower than the lateral malleolus towards a position which lies higher than the medial malleolus. The draw strips are connected to the top section of the support element. The draw strips can be directly connected thereto, for example by means of press-studs, buckles or Velcro fasteners. An eminently suitable and preferred manner of connection consists in the draw strips running upwards along the lower leg, being passed through a slot-like opening in the top section of the support element and then being turned back, after which ultimately the turned-back section is fixed to the incoming part, for example by means of Velcro fastenings. This makes fitting very easy and provides a great deal of flexibility in terms of adaptation to the dimensions of the foot and lower leg, which differ from person to person. If desired, it is possible for a plurality of draw strips to be fitted, but they run substantially as indicated for the first two.

To fix the support element against movement in its longitudinal direction, it can be attached, for example, by means of strips around the lower leg. Fixing means which are preferred are formed by a sole section which runs virtually perpendicular to the support element and, with the ankle support fitted, runs below the sole of the foot, preferably anatomically adjoining it. In this way, a movement of the support element in the upward direction along the lower leg is prevented since the sole section is held in place by the sole of the foot and a movement in the downward direction is prevented by the supporting action of the sole section on the ground or on the inside of a shoe, which under normal circumstances is worn over the ankle support.

A unit which is held together even in the state in which it is not being worn and is simple to fit is advantageously obtained if that section of the securing element which runs beneath the foot is provided with a pocket in which the sole section can be accommodated.

The ankle support according to the invention may also be equipped with a sock, which if desired leaves the front of the foot and/or the heel exposed, to which the securing element is attached and to which the support element is also attached, preferably movably. The sock holds the various elements of the ankle support according to the invention at the desired relative position and makes it easy to fit the ankle support.

If desired, it is also possible for pressure-applying bandages to be attached to the sock, which bandages can be attached around the ankle joint, for example using Velcro fastenings.

The invention will be explained with reference to the following drawings, in which:
Fig. 1 shows a perspective view, on an angle and from the front, of the lateral side of a foot on which an ankle support according to the invention is fitted:
Fig. 2 shows a perspective view, on an angle from the rear, of the foot shown in Fig. 1; and
Fig. 3 shows a view of the foot from below.

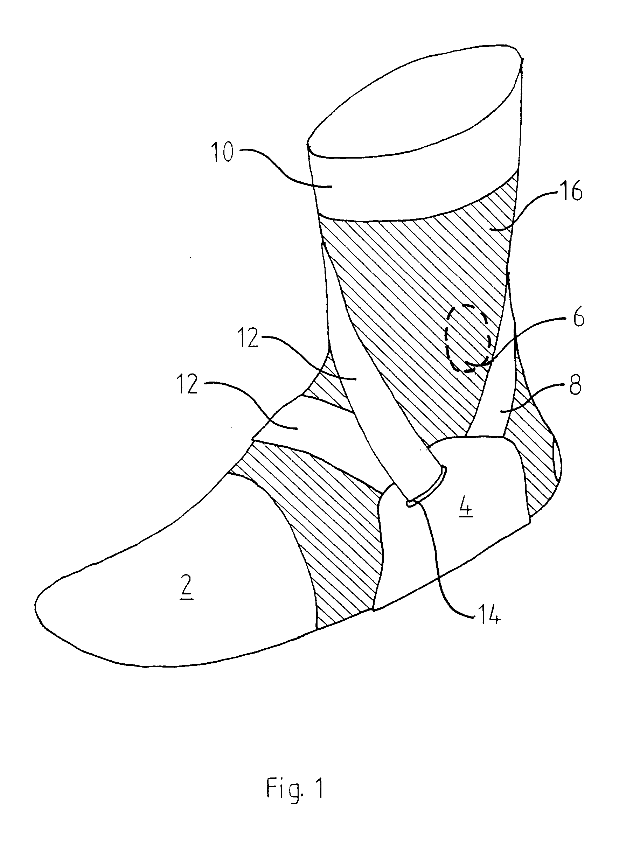

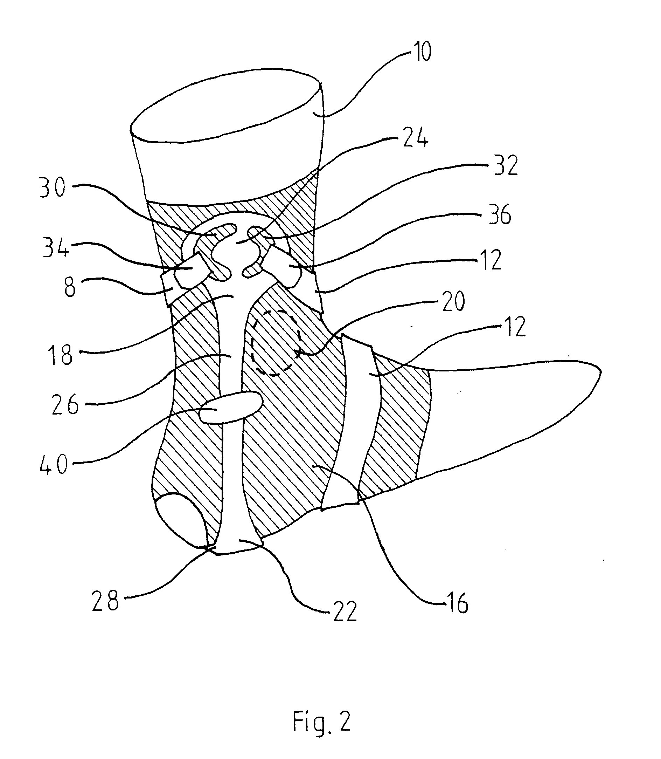

Figs. 1 and 2 show a foot which is adjoined by a securing element 4, at the lateral section of the foot, which lies below the lateral malleolus 6. Draw strip 8 is attached to the highest section of the securing element and, from there, runs obliquely upwards along the rear side of the lower leg 10.

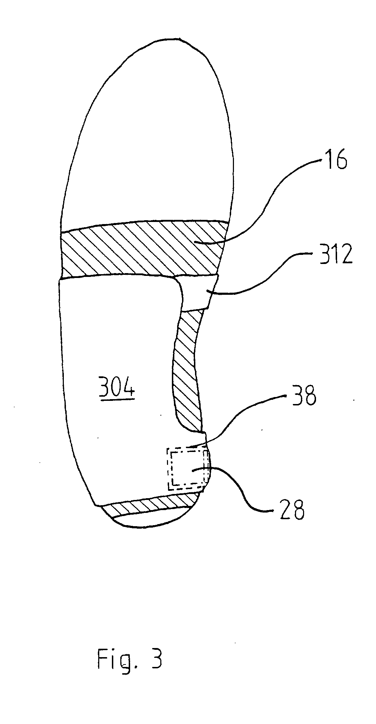

A second draw strip 12 is attached to the opposite side of the securing element, as can be seen from Fig. 3 (this securing is not visible in Fig. 1) and, from the medial side of the foot, runs over the instep, through a slot 14 in securing element 4. There, draw strip 12 is turned back and then runs obliquely upwards in front of the lower leg 10, towards the medial side of the lower leg 10. The hatching indicates a sock 16 which encloses the lower leg 10 and part of the foot 2 and to which securing element 4 is attached.

In Fig. 2, a support element 18 runs upwards from a position below the medial malleolus 20, along the lower leg 10. The element 18 comprises a bottom section 22, a top section 24 and a connecting section 26. A sole section 28, which runs beneath the foot, is present at the bottom section 22. From the lateral side, draw strip 8 runs obliquely upwards along the rear side of the lower leg 10 and runs towards the top section 24 of support element 18. Along the front side of the lower leg, draw strip 12 runs in a similar way towards the top section 24. Two slots 30 and 32 are present in the top section 24. The draw strips 8 and 12 have been pulled through slots 30 and 32, respectively, from below and have been folded back towards the front. The strips 8 and 12 have been pulled tight and the folded-back ends 34 and 36 have been secured by means of Velcro fastenings (not visible) to that part of draw strips 8 and 12 which runs upwards from the lateral side. The medial section, which functions as a closure strip, of draw strip 12 runs past the front of the medial malleolus 20, over the instep, in the direction of the slot 14 in securing element 4 which is illustrated in Fig. 1. Draw strip 12 is secured to securing element 4 beneath the foot, as can be seen in Fig. 3. The support element runs through a strip 40 which is stitched onto the sock 16 and is thus movably connected to the sock 16.

In Fig. 3, 304 denotes that section of securing element 4 which runs beneath the foot. 312 denotes that end of draw strip 12 which is secured to the securing element 304 beneath the foot. The dashed line 38 denotes a pocket in the securing element 304 in which a sole section 28, which is indicated by a dot-dashed line, of the support element is accommodated.

## Claims

1. Ankle support having a lateral side and a medial side, at least comprising
- an elongate support element (18), which runs on the medial side, has a bottom section (22) and a top section (24) and is anatomically adapted to the lower leg (10), the ankle and the foot, which support element (18) is provided with means for fixing it against movement in its longitudinal direction, **characterized in that** the ankle support also comprises:
- a securing element (4) which runs on the lateral side;
- at least two draw strips (8, 12) which, when the ankle support is arranged on the foot, each run along different sides of the lower leg (10) and connect the top section (24) of the support element (18) to the securing element (4).

2. Ankle support according to Claim 1, in which there is a closure strip for fixing the securing element (4) with respect to the foot (2).

3. Ankle support according to Claim 2, in which the securing element (4) continues under the sole of the foot.

4. Ankle support according to Claim 2 or 3, in which the closure strip forms a single unit with one of the draw strips.

5. Ankle support according to one of Claims 1-4, in which the support element (18) is provided, at the location of the bottom section (22), with a virtually flat sole section (28).

6. Ankle support according to Claim 5, in which the securing element (18), in the section (304) which continues under the foot, has a pocket (28) in which the sole section (28) can be accommodated.
